# EUROPEAN PATENT SPECIFICATION

(11) **EP 2 334 409 B1**
(45) Date of publication and mention of the grant of the patent: **19.09.2012**
(21) Application number: 09760028.2
(22) Date of filing: 05.10.2009
(51) Int. Cl.: B01D 59/44, C07C 51/16, C07C 51/41, C12P 7/06, G01N 33/14

(54) **METHOD FOR DETERMINATION OF DELTA-D VALUES OF NON- EXCHANGEABLE HYDROGEN STABLE ISOTOPES ON ETHANOL' S METHYL GROUP BY MEANS OF IRMS INSTRUMENTAL TECHNIQUE**
VERFAHREN ZUR BESTIMMUNG VON DELTA-D-WERTEN VON NICHT AUSTAUSCHBAREN STABILEN WASSERSTOFFISOTOPEN AN DER METHYLGRUPPE VON ETHANOL MITTELS DER IRMS-INSTRUMENTALTECHNIK
PROCÉDÉ POUR LA DÉTERMINATION DE VALEURS DELTA-D D'ISOTOPES STABLES D'HYDROGÈNE NON ÉCHANGEABLES SUR LE GROUPE MÉTHYLE DE L'ÉTHANOL AU MOYEN D'UNE TECHNIQUE INSTRUMENTALE IRMS

(30) Priority: 06.10.2008 RS 8046208
(43) Date of publication of application: 22.06.2011
(73) Proprietor: Smajlovic, Ivan, 21220 Becej (RS)
(72) Inventor: Glavanovic, Milanka, 26000 Pancevo (RS); Smajlovic, Ivan, 21220 Becej (RS)
(86) International application number: PCT/RS2009/000023
(87) International publication number: WO 2010/041971

(56) References cited:
- ROSSMANN A ET AL: "ASSIGNMENT OF ETHANOL ORIGIN AND PROOF OF SUGAR ADDITION TO WINE THROUGH POSITIONAL DEUTERIUM AND CARBON-13 ISOTOPE RATIO MEASUREMENT" ZEITSCHRIFT FUER LEBENSMITTELUNTERSUCHUNG UND -FORSCHUNG, XX, XX, vol. 188, no. 5, 1 January 1989 (1989-01-01), pages 434-438, XP009128982 ISSN: 0044-3026
- RAUSCHENBACH P ET AL: "COMPARISON OF THE DEUTERIUM AND CARBON-13 CONTENTS OF ETHANOL OBTAINED BY FERMENTATION AND CHEMICAL SYNTHESIS" ZEITSCHRIFT FUER NATURFORSCHUNG. C, A JOURNAL OF BIOSCIENCES, TUEBINGEN, DE, vol. 34, no. 1-2, 1 January 1979 (1979-01-01), pages 1-4, XP009128963 ISSN: 0939-5075
- DEPPENMEIER U ET AL: "Biochemistry and biotechnological applications of Gluconobacter strains" APPLIED MICROBIOLOGY AND BIOTECHNOLOGY, SPRINGER VERLAG, BERLIN, DE, vol. 60, no. 3, 1 November 2002 (2002-11-01), pages 233-242, XP002334995 ISSN: 0175-7598
- TESFAYE W ET AL: "Wine vinegar: technology, authenticity and quality evaluation" TRENDS IN FOOD SCIENCE AND TECHNOLOGY, ELSEVIER SCIENCE PUBLISHERS, GB, vol. 13, no. 1, 1 January 2002 (2002-01-01), pages 12-21, XP004376502 ISSN: 0924-2244
- NIGEL SAUNDERS: "Oxidation of ethanol to ethanoic acid"[Online] 20 April 2004 (2004-04-20), pages 1-2, XP002568402 www.creative-chemistry.org Retrieved from the Internet: URL:http://web.archive.org/web/20040420080 512/http://creative-chemistry.org.uk/aleve l/module3/documents/N-ch3-15.pdf> [retrieved on 2010-02-12]
- BENSON S ET AL: "Forensic applications of isotope ratio mass spectrometry-A review" FORENSIC SCIENCE INTERNATIONAL, ELSEVIER SCIENTIFIC PUBLISHERS IRELAND LTD, IE, vol. 157, no. 1, 10 February 2006 (2006-02-10), pages 1-22, XP025086081 ISSN: 0379-0738 [retrieved on 2006-02-10]

## Description

### Technical field

The present innovation is related with chemical instrumental analysis, i.e. to the domain of analysis of stable isotopes in food products, and it is related to the method for preparation of ethanol samples and mode for determination of isotopic ratio of non-exchangeable hydrogen stable isotopes sited on the methyl site of ethanol by means of instrumental technique CF - TC/EA - IRMS (Continuous Flow - Temperature Conversion/Elemental Analyzer - Isotope Ratio Mass Spectrometry), and for the purpose of establishing authenticity and geographical origin of wine and grape must, beer, alcoholic beverages, fruit juices, honey, vinegars and other food products which contain alcohol and /or fermentable sugars.

### Background art

Isotopic methods have shown that they can be very powerful analytical tool for authenticity and geographical origin determination of wines and strong spirits. By measuring the content of stable isotopes in these products useful information can be provided for detection of many frauds in wine and strong spirits production. Instrumental techniques which are used for isotopic measurements are based on measuring the relative ratios of stable isotopes by means of Isotope Ratio Mass Spectrometry.

Systems comprising a pyrolysis chamber and continuous flow isotope spectrometer CF - TC/EA - IRMS (Flash HT Continuous Flow - Temperature Conversion/Elemental Analyzer - Isotope Ratio Mass Spectrometry) are commercially available for stable hydrogen analysis of solid and liquid samples.

When analyzing ethanol samples, by means of CF - TC/EA - IRMS (Continuous Flow - Thermal Conversion/Elemental Analyzer - Isotope Ratio Mass Spectrometry), because of the ethanol's hydroxyl group, which contains easily exchangeable hydrogen, gained δD values for ethanol of the same botanical and geographical origin can vary, and for that reason it is impossible to perform qualitative and quantitative identification of the ethanol sample origin.

One of the problems which can occur, for example, in strong spirit production, is in finalization production steps. The distillate is diluted with water to determined alcoholic strength which is necessary so that alcoholic drink could be consumed. By adding water which has different isotopic content, dynamic isotopic equilibrium is disturbed and hydrogen or deuterium which is bonded to oxygen atom of the hydroxyl group is exchanged. This is one of the reasons for gaining the wrong δD values and wrong information about ethanol origin.

Because of the problems which are stated above, instrumental technique CF - TC/EA - IRMS can not be very useful in detection of frauds in wine and alcoholic drinks production and moreover for detection of ethanol which originates from beet sugar, barley, wheat etc. in wine and alcoholic drinks.

According to the first aspect of this invention, the method for preparation of ethanol samples and mode for the determination of isotopic ratio of non-exchangeable hydrogen stable isotopes sited on the methyl site of ethanol by means of instrumental technique CF - TC/EA - IRMS (Continuous Flow - Temperature Conversion/Elemental Analyzer - Isotope Ratio Mass Spectrometry) is based on full enzymatic (or organic) transformation of ethanol samples to gain ethanoic acid (acetic acid), controlled neutralization of acetic acid and further concentration, purification of prepared acetate salt and its drying to the constant mass and further determination of δD values in prepared samples by means of CF-TC/EA-IRMS.

### Background Art

Deuterium and hydrogen relative ratio measurements for the proposal of authenticity and origin determination of wines and alcoholic drinks, beer, fruit juices and honey today is done by means of NMR methodology (Site Natural Isotopic Fractionation - Nuclear Magnetic Resonance) which is based on intermolecular scanning of the measured ethanol sample and determination of isotopic composition of hydrogen and deuterium atoms sited on the first and on the second carbon atom inside of the ethanol molecule. Results gained by NMR methodology give the information about the presence of ethanol which originates from beet sugar or other industrial plants, and which belongs to the C3 plant group.

This instrumental technique has limited use in practice, because it can be used only if database for wine samples is previously made and that without specific database conclusions can be very subjective and quantitative analysis hard. For that reason it is used only for wine with designated geographical origin for which databases are previously made. Other lacks of this instrumental technique are:
- NMR methodology is very time consuming method
- It requires big consumption of helium and liquid nitrogen and also electrical energy,
- It occupies big part of the working place because of it's size and because of the very strong magnetic field which it makes (security zone is needed),

### Disclosure of invention

The main goal of this invention is to overcome the barrier and lacks of today known apparatus and methods for determination of isotopic composition of non-exchangeable hydrogen and deuterium atoms in ethanol samples.
According to the invention, the method for preparation of ethanol samples and mode for the determination of isotopic ratio of non-exchangeable hydrogen stable isotopes sited on the methyl site of ethanol (CH₃- group) is based on the full enzymatic transformation of ethanol samples to acetic acid (ethanoic acid) in presence of enzymes ethanol-dehydrogenase and acetaldehyde-dehydrogenase and coenzyme NAD⁺, controlled neutralization of gained acetic acid to make acetate, purification of prepared acetate and vaporization to the constant mass and further determination of δD values in prepared samples by means of CF-TC/EA-IRMS.

Conversion of ethanol into acetic acid alternatively can be done by organic oxidation of ethanol in presence of sodium bichromate (IV). This aspect is however not part of the invention.

Chemical (as opposed to enzymatic) conversion of ethanol to acetic acid for the purpose of measuring δD values is disclosed in the following documents:
ROSSMANN A ET AL: "Assignment of ethanol origin and proof of sugar addition to wine through positional deuterium and carbon-13 isotope ratio measurement", Zeitschrift fuer Lebensmitteluntersuchung und -forschung, vol. 188, no. 5, 1989, pp. 434-438, ISSN: 0044-3026;
RAUSCHENBACH P ET AL: "Comparison of the deuterium and carbon-13 contents of ethanol obtained by fermentation and chemical synthesis", Zeitschrift fuer Naturforschung, vol. 34c, no. 1-2, 1979, pp. 1-4, ISSN: 0939-5075.

According to a further aspect of this invention, method for preparation of ethanol samples and their conversion into acetic acid alternatively can be done by recirculation previously isolated ethanol sample through the continuous flow column with mixture of two immobilized enzymes ethanol-dehydrogenase and acetaldehyde-dehydrogenase and in presence of coenzyme NAD⁺.

### Best Modes for Carrying Out of the Invention

According to the invention, invention, the method for preparation of ethanol samples and mode for the determination of isotopic ratio of non-exchangeable hydrogen stable isotopes sited on the methyl site of ethanol (CH₃- group) is based on the full enzymatic transformation of ethanol samples to acetic acid (ethanoic acid) in presence of enzymes ethanol-dehydrogenase and acetaldehyde-dehydrogenase and coenzyme NAD⁺, controlled neutralization of gained acetic acid to make acetate, purification of prepared acetate and vaporization to the constant mass and further determination of δD values in prepared samples by means of CF-TC/EA-IRMS.
First step of the process is based on the isolation of ethanol from wine, strong spirits, fermented fruit juices fermented honey solutions samples by distillation. Prior to distillation it is needed to neutralize all volatile acids in the sample which could past over into the distillate.
Second step is based on the full enzymatic transformation of previously isolated ethanol to produce acetic acid without isotopic fractionation. This is done in presence of two specific enzymes, ethanol dehydrogenase and acetaldehyde dehydrogenase and oxido-reduction coenzyme NAD⁺.
Third step involves isolation of gained acetic acid from the reaction mixture without any quantitative lost by distillation in presence of overheated water vapors or by vacuum distillation.
Forth step involves neutralization of isolated acetic acid with Sodium Hydroxide or some other alkali base, Sodium Carbonate or similar, in presence of pH-meter until pH value of 8,1 and in that way preparation of acetate salt (preferable Sodium Acetate).
Step five involves concentrating of acetate solution by means of vacuum distillation until the solution gets syrupy consistence. The distillate can be rejected.
Sixth step involves purification of cooled Sodium Acetate with diethyl ether to remove all organic impurities which are lapsed n the matrix. It can be by using ultrasonic water bath and then removal of the ether phase with help of the separation glass. This step should be repeated three times. After purification vaporization of Sodium acetate can be done to dry and constant mass.

Further step involves determination of the relative ratio of hydrogen stable isotopes in prepared Sodium Acetate sample which comes from un-exchanged methyl group inside of acetate salt.

According to a further aspect of this invention, at the beginning of the method, prior to distillation and isolation of ethanol from wine, strong spirits, fermented fruit juices or fermented honey solutions samples, they must be neutralized to avoid isotopic fractionation and further loss during the distillation step by addition of sodium hydroxide solution (NaOH) until pH value reach 8,1 to 8,5. It that way all present organic acids will be neutralized and present carbonyl compounds - aldehydes and ketons will be retrogressed. In this way after distillation, gained distillate is acids free and further process steps are the same as described prior in the text.

Also, according to a further aspect of this invention second step of the method which involves ethanol conversion to acetic acid can be carried out by recirculation of previously isolated neutralized ethanol from analyzed samples, in presence of NAD⁺ trough the column with immobilized mixture of ethanol-dehydrogenase and acetaldehyde dehydrogenase enzymes and for the purpose of faster biochemical conversion of ethanol into acetic acid.

In accordance with the idea of this invention, the method for preparation of ethanol samples and mode for the determination of isotopic ratio of non-exchangeable hydrogen stable isotopes sited on the methyl site of ethanol (CH₃- group), and for the purpose of authenticity and geographical origin determination of wines and grape musts, beers, alcoholic drinks, fruit juices, honey and all other food products which contain alcohol and/or fermentable sugars, it has it's advantages:
- In the first place, it gives very good precision and repeatability of results for δD values of analyzed ethanol samples, no matter if ethanol sample was diluted with water before distillation, and it gives espied constant difference and dependence between ethanol samples with botanical origin from C3 group of plants;
- There is no need for big financial funds and special conditions for maintenance like which is the case with instrumental technique NMR methodology,
- It gives the opportunity to detect the presence of ethanol which originates from beet sugar, wheat, barley and other industrial plants which belong to the C3 group of plants, in the ethanol samples which are isolated from the analyzed wines and alcoholic drinks or fermented juices and fermented honey.

It will be appreciated that modifications to the embodiments described above are of course possible. The present innovation is limited only by the scope of the appended claims.

### Industrial Applicability

The method for preparation of ethanol samples and mode for the determination of isotopic ratio of non-exchangeable hydrogen stable isotopes sited on the methyl site of ethanol (CH₃- group) is applicable in instrumental analytical chemistry and is used for authenticity and geographical origin determination of wines and grape musts, alcoholic drinks, beers, fruit juices, honey and other food products which contain ethanol and/or fermentable sugars.

## Claims

1. A process for determining origin of an alcohol-containing food product, comprising:
providing a sample of the alcohol-containing food product; neutralizing volatile acids in the provided sample;
degrading carbonyl compounds: aldehydes and ketones in the provided sample; extracting an ethanol sample from the provided sample;
providing enzymes ethanol dehydrogenase and acetaldehyde dehydrogenase, and a oxido-reduction coenzyme NAD⁺ to convert ethanol in the extracted ethanol sample to acetic acid;
extracting the acetic acid, wherein the step of extracting the acetic acid includes at least one of the following processes: distillation in the presence of overheated water vapor, or vacuum distillation;
producing an acetate salt solution from the extracted acetic acid by neutralizing the extracted acetic acid until the pH value of solution reaches 8.1;
concentrating the produced acetate salt solution by vacuum distillation until the acetate salt solution reaches syrupy consistency, wherein a byproduct distillate is discarded; cooling the concentrated acetate salt solution;
removing impurities from the concentrated and cooled acetate salt solution by adding an organic solvent and mixing the solution in an ultrasonic water bath, wherein the organic solvent dissolves the impurities;
discarding the organic solvent containing the dissolved impurities using a separation glass;
obtaining acetate salt dried to constant mass from the purified concentrated acetate salt solution;
measuring an, isotopic composition, i.e., isotopic relative ratio of deuterium and hydrogen of the dried acetate salt;
calculating a δD value for the measured isotopic composition; and
comparing the calculated δD value with δD values associated with a food product of known origin.

2. The process of claim 1, wherein the step of neutralizing volatile acids includes adding Sodium Hydroxide solution until pH value of the mixture reaches value between 8.1 and 8.5.

3. The process of claim 1, wherein the step of producing an acetate salt solution from the extracted acetic acid by neutralizing the extracted acetic acid until the pH value of solution reaches 8.1, includes using an alkaline base.

4. The process of claim 3, wherein the alkaline base is Sodium Hydroxide.

5. The process of claim 1, wherein the step of producing an acetate salt solution from the extracted acetic acid by neutralizing the extracted acetic acid until the pH value of solution reaches 8.1, includes using a weak acid salt.

6. The process of claim 5, wherein the weak acid salt is Sodium Carbonate.

7. The process of claim 1, wherein the organic solvent used in the steps of removing impurities from the concentrated and cooled acetate salt solution, and discarding the organic solvent containing the dissolved impurities, includes a diethyl ether.

8. The process of claim 1, wherein the steps of removing impurities from the concentrated and cooled acetate salt solution, and discarding the organic solvent containing the dissolved impurities, are repeated three times.

9. The process of claim 1, further comprising:
providing a column with an immobilized mixture of ethanol dehydrogenase and acetaldehyde dehydrogenase enzymes;
andrecirculating the ethanol sample in presence of oxido-reduction coenzyme NAD⁺ through the provided column.

## Patentansprüche

1. Ein Verfahren für die Bestimmung des Ursprungs einer alkoholhaltigen Lebensmittel, beinhaltet:
die Bereitstellung eines Musters von der alkoholhaltigen Lebensmittel; neutralisierende volatile Säuren ins mitgelieferte Muster;
degradierende Carbonylgruppen: Aldehyde und Ketone ins mitgelieferte Muster; die Gewinnung von Ethanoimuster aus dem mitgelieferten Muster;
die Besorgung der Enzyme Ethanol Dehydrogenase und Acetaldehyde Dehydrogenase, und Redoxreaktion Coenzyme NAD⁺, um Ethanol in dem extrahierten Muster zur Essigsäure umzuwandeln;
die Extraktion der Essigsäure, worin der Schritt von der Besorgung der Essigsäure mindestens einen von den folgenden Prozessen einschließt: die Destillation in der Anwesenheit von dem überhitzten Wasserdampf, oder der Vakuumdestillation;
die Herstellung von der Acetate Salzlösung von der extrahierten Essigsäure, indem man die extrahierte Essigsäure neutralisiert, bis der pH Wert von der Lösung 8.1 erreicht;
die gewonnene Acetate Salzlösung wird von dem Vakuumdestillation konzentriert, bis Acetate Salzlösung sirupartige Zusammensetzung erreicht, und das Nebenprodukt Destillat wird abgelegt; die Abkühlung der Acetate Salzlösung;
die Entfernung von Verunreinigungen aus der konzentrierten und gekühlten Acetate Salzlösung wird durch Zugabe eines organischen Lösungsmittels und Mischung der Lösung im Ultraschallbad mit destilliertem Wasser durchgeführt, worin das organische Lösungsmittel die Verunreinigungen löst;
die Entfernung des organischen Lösungsmittels, die die gelösten Verunreinigungen hat, mithilfe eines Trennungsglases;
die Gewinnung Acetate Salz getrocknet zur konstanten Masse aus der reinigten konzentrierten Salzlösung;
die Messung der Isotopenzusammensetzung, d. h., das isotopische relative Verhältnis von Deuterium und Wasserstoff von dem getrockneten Acetat Salz;
Berechnung vom δD Wert für die gemessene isotopische Zusammensetzung; und
der Vergleich vom berechneten δD Wert mit δD Werte, die mit einem Lebensmittel bekannter Herkunft die Verbindung hat.

2. Der Anspruchsprozess 1, worin der Schritt der Neutralisation der flüchtigen Säuren, das Hinzufügen Natronlauge bis der pH-Wert des Gemisches den Wert zwischen 8.1 und 8.5 erreicht, beinhaltet.

3. Der Anspruchsprozess 1, worin der Schritt der Erzeugung Acetate Salzlösung von derextrahierten Essigsäure, indem man die extrahierte Essigsäure neutralisiert, bis der pH Wert der Lösung 8.1 erreicht, auch den Gebrauch von Basen einschließt.

4. Der Anspruchsprozess 3, worin die Base Natriumhydroxid ist.

5. Der Anspruchsprozess 1, worin der Schritt der Erzeugung Acetate Salzlösung von der extrahierten Essigsäure, indem man die extrahierte Essigsäure neutralisiert, bis der pH Wert der Lösung 8.1 erreicht, auch den Gebrauch von der schwachen Essigsäure einschließt.

6. Der Anspruchsprozess 5, worin die schwache Essigsäure Natriumkarbonat ist.

7. Der Anspruchsprozess 1, worin das organische Lösungsmittel für die Schritte zur Entfernung von Verunreinigungen aus der konzentrierten und gekühlten Acetat Salzlösung, und Abwerfen des organischen Lösungsmittels, das die gelösten Verunreinigungen enthält, enthält einen Diethylether.

8. Der Anspruchsprozess 1, worin die Schritte zur Entfernung von Verunreinigungen aus der konzentrierten und gekühlten Acetat Salzlösung, und Abwerfen des organischen Lösungsmittels, das die gelösten Verunreinigungen enthält, dreimal wiederholt werden.

9. Der Anspruchsprozess 1, fasst weiter um:
Gibt eine Spalte mit der immobilisierten Mischung von Ethanol Dehydrogenase und
Enzyme der Acetaldehyd Dehydrogenase; und rezirkuliert das Ethanolmuster in der Anwesenheit von der Oxido - Reduktion Coenzym NAD⁺ durch die gegebene Spalte.

## Revendications

1. Le procès pour déterminer l'origine d'un produit alimentaire contenant de l'alcool comprend :
- assurer un échantillon d'un produit alimentaire contenant de l'alcool
- neutraliser l'acide volatil dans l'échantillon assuré.
- extraire l'échantillon d'éthanol de l'échantillon assuré.
- dégrader les composes carbonyles-les aldéhydes et les cétones-de l'échantillon assure.
- assurer les enzymes -éthanol déshydrogénase, acétaldéhyde déshydrogénase et coenzyme d'oxydoréduction NAD+ pour que l'éthanol de l'échantillon se convertisse a l'acide acétique.
- extraire de l'acide acétique en utilisant au moins l'un des procès suivants : Une distillation en présence de vapeur surchauffée ou une distillation vide
- produire une solution d'acétate de sodium d'une acide acétique extracte jusqu'à ce que la valeur PH atteigne 8.1.
- concentrer la solution produite de l'acétate de sodium par la distillation vide jusqu' à ce que la solution de l'acétate de sodium atteigne la consistance de sirop ou le sous-produit distille est rejeté.
- refroidir la solution concentrée d'acétate de sodium
- rejeter les impuretés de l'acétate de sodium concentre et refroidi en y ajoutant un solvant organique ou en mélangeant la solution dans un bain a ultrasons
- rejeter le solvant organique contenant des impuretés dissolues en utilisant verrerie.
- obtenir d'une solution d'acétate de sodium purifié et concentre une masse constante d'acétate de sodium sèche
- mesurer une composition isotopique par exemple : ratio relative des isotopes de l'hydrogène dans l'acétate de sodium sec
- calculer la valeur δD de la composition isotopique mesurer et comparer la valeur D calculée avec les valeurs δD qui sont associées au produit alimentaire dont l'origine est connu.

2. Le procès d'attestation 1. ou la neutralisation de l'acide volatile comprend l'ajoutage de la solution d'hydroxide de sodium jusqu'à la valeur PH soit entre 8.1 et 8.5

3. Le procès d'attestation 1.ou la production de solution d'acétate d'acide d'une acide acétique extractée en utilisant la neutralisation de l'acide acétique jusqu'à PH 8.1 inclut l'utilisation d'une base alcaline

4. Le procès d'attestation 3. ou la base alcaline est l'hydroxide de sodium

5. Le procès d'attestation 1. Ou la production de solution d'acétate de sodium de l'acide acétique par la neutralisation de l'acide acétique extracte jusqu' a PH valeur de la solution atteigne 8.1 inclut l'usage de l'acide de sodium faible.

6. Le procès d'attestation 5., ou l'acide de sodium faible est le carbonate de sodium.

7. Le procès d'attestation 1.ou le solvant organique utilise lors du rejet des impuretés de la solution d'acide de sodium refroidie et concentrée et le rejet d'un solvant organique contenant des impuretés, inclut l'éther diethilique.

8. Le procès d'attestation 1. Ou le rejet des impuretés de la solution d'acétate acide concentrée et refroidie et le rejet d'un solvant organique contenant les impuretés dissolues sont répétés trois fois.

9. Le procès d'attestation 1. Comprend plus loin :
l'assurance d'une colonne avec une mixture immobilisée des enzymes-éthanol déshydrogénase et acétaldéhyde déshydrogénase et la recirculation de l'échantillon de l'éthanol en présence de coenzyme d'oxydoréduction NAD+ a travers de la colonne assurée.
